(19) Europäisches Patentamt
European Patent Office
Office européen des brevets

(11) Veröffentlichungsnummer: **0 110 116**
**B1**

(12) # EUROPÄISCHE PATENTSCHRIFT

(45) Veröffentlichungstag der Patentschrift: **04.01.89**

(51) Int. Cl.[4]: **C 07 D 249/08,** C 07 D 233/60, C 07 D 235/06

(21) Anmeldenummer: **83110592.9**

(22) Anmeldetag: **24.10.83**

(54) **Verfahren zur Herstellung von Keten-O,N-acetalen.**

(30) Priorität: **30.10.82 DE 3240288**

(43) Veröffentlichungstag der Anmeldung: **13.06.84 Patentblatt 84/24**

(45) Bekanntmachung des Hinweises auf die Patenterteilung: **04.01.89 Patentblatt 89/1**

(84) Benannte Vertragsstaaten: **AT BE CH DE FR GB IT LI NL SE**

(56) Entgegenhaltungen:
**EP-A-0 056 125**
**EP-A-0 106 322**
**FR-A-1 486 817**

**HOUBEN-WEYL, Methoden der Organischen Chemie, Bd VII/4, 1968, S. 362-64**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Ruland, Alfred, Dr., Schriesheimer Strasse 11, D-6945 Hirschberg (DE)**
Erfinder: **Reuther, Wolfgang, Dr., Am Pferchelhang 16, D-6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 110 116 B1

LIBER, STOCKHOLM 1989

**Beschreibung**

Die vorliegende Erfindung betrifft ein Verfahren zur Herstellung von Keten-O,N-acetalen durch Umsetzung der entsprechenden Keten-O,O-acetale mit einer heterocyclischen Stickstoffverbindung.

Keten-O,N-acetale und ihre Anwendung als Fungizide sind bekannt (EP-56 125). Sie werden hergestellt durch Zersetzung der entsprechenden Phenylsulfonate bei 10 bis 100°C in Gegenwart eines Lösungsmittels und eines Katalysators. Die Umsetzung von Keten-O,O-acetalen mit Aminen ist bekannt (HOUBEN WEYL, Methoden der Organischen Chemie, Band VII/4, 1968, S. 362 bis 364).

Diese Herstellungsverfahren haben den Nachteil, daß die Keten-O,N-acetale als Mischung der entsprechenden E- und Z-Isomeren anfallen. Für den Fall, daß nur eines der beiden Isomeren von Interesse ist müssen die Isomeren getrennt werden und es entsteht ein Zwangsanfall an dem uninteressanten Isomeren.

Es wurde nun gefunden, daß man Keten-O,N-acetale der Formel I

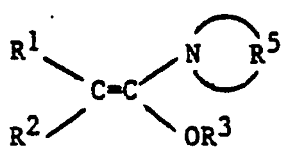

I

in der

$R^1$ einen tertiären Alkylrest mit 4 bis 6 C-Atomen

$R^2$ Wasserstoff,

$R^3$ einen gegebenenfalls durch Halogen, Alkoxy, Alkyl, Phenyl, Phenoxy, Cyano, Nitro, Trifluormethyl, substituierten Phenylrest und

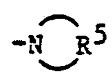

einen Triazol-, Pyrazol-, Benzimidazol- oder Imidazolrest

bedeutet, erhält, wenn man ein Keten-O,O-acetal der Formel II

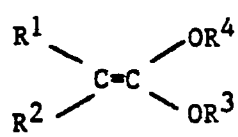

II

in der

$R^1$, $R^2$ und $R^3$ die oben genannten Bedeutungen haben und

$R^4$ einen gegebenenfalls durch Halogen, Alkoxy($C_1$-$C_2$), Alkyl($C_1$-$C_4$), Phenyl oder Phenoxy, Cyano, Nitro, Trifuormethyl substituierten Phenylrest bedeutet, bei einer Temperatur von 100 bis 250°C mit der stöchiometrischen Menge oder bis zu 10 % mehr oder weniger einer Verbindung der Formel

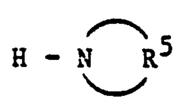

umsetzt, in der

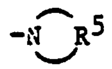

die oben genannten Bedeutungen hat.

$R^1$ bedeutet beispielsweise einen tertiär-Butylrest.

$R^3$ bedeutet beispielsweise einen durch Halogen (F, Cl, Br), Alkoxy mit 1 bis 2 C-Atomen (Methoxy), Alkyl mit 1 bis 4 C-Atomen (Methyl, Ethyl, Propyl, i-Propyl, Butyl, sek.-Butyl, tert.-Butyl, i-Butyl) substituierten Phenylrest, wobei einzelne Reste einfach oder mehrfach (2-fach) oder verschiedene Reste gleichzeitig als Substituenten auftreten, z. B. 4-Chlorphenyl, 3,4-Dichlorphenyl, 4-Chlor-3-bromphenyl, 4-Methyl-3-chlorphenyl.

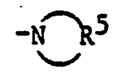

bedeutet beispielsweise 1-(1,2,4)-Triazolyl, 1-(1,2,3-Triazolyl).

Erhöhte Temperatur bedeutet beispielsweise eine Temperatur höher als 80° C.

Nach dem erfindungsgemäßen Verfahren wird im Gegensatz zum bekannten Verfahren ein Isomeres (E- oder Z-Isomeres) in fast quantitativer Ausbeute und einer Reinheit von 75 bis 95 % erhalten. Die weitgehend reinen Isomeren können leicht in üblicher Weise (Umkristallisation, Adsorption an Adsorptionsmitteln) gereinigt und so die vollständig reinen Isomeren erhalten werden. In Abhängigkeit von der Konstitution der Ausgangsprodukte entsteht bei der Umsetzung entweder das E- oder Z-Isomere. Beim bekannten Verfahren liegen die als Ausgangsprodukte verwendeten Phenylsulfonate in Form von Diastereomerengemischen vor, weil sie zwei asymnetrische Kohlenstoffatome enthalten. Bei der bekannten Zersetzung dieser Diastereomerengemische der Phenylsulfonate entsteht ein Gemisch der E- und Z-isomeren Verbindungen. Nachteilig beim bekannten Verfahren ist die zum Teil umständliche und schwierige Trennung der Diastereomerengemische vor der Zersetzung und der damit verbundene Zwangsanfall des unerwünschten Isomeren, der mit einer zum Teil starken Verminderung der Gesamtausbeute einhergeht. Dieser Nachteil ergibt sich auch, wenn die Isomerentrennung erst nach der Zersetzung erfolgt.

Das erfindungsgemäße Verfahren kann in Gegenwart von Lösungsmitteln oder vorteilhaft in Abwesenheit eines Lösungsmittels unter erhöhtem Druck oder vorteilhaft bei Normaldruck durchgeführt werden. Wesentlich ist, daß die für die Umsetzung notwendige erhöhte Temperatur, vorzugsweise 180 bis 200° C erretcht wird. Bei der Umsetzung geht der Rest -$OR^4$ in das entsprechende Phenolderivat $HOR^4$ über. Im Laufe der Umsetzung entstehen steigende Mengen an diesem Phenolderivat. Es stört die Umsetzung nicht und muß daher nicht laufend aus der Umsetzungsmischung entfernt werden.

Nach Beendigung der Umsetzung kann das entstandene Phenolderivat in üblicher Weise beispielsweisc durch Überführung in das entsprechende wasserlösliche Alkaliphenolat von dem Endprodukt oder seiner Lösung in einem organischen Lösungsmittel abgetrennt werden.

Die Umsetzung kann beispielsweise mit einem Überschuß eines Reaktionsteilnehmers (z. B. der Verbindung H-N $R^5$, insbesondere 1,2,4-Triazol) über die stöchiometrische Menge (z. B. bis zu 10 % Überschuß) durchgeführt werden. Vorzugsweise werden stöchiometrische Mengen der Ausgangsprodukte verwendet.

Die als Endprodukte der Umsetzung erhaltenen weitgehend reinen Isomeren können ohne weitere Reinigung als Fungizide verwendet werden. Die als Ausgangsprodukte benötigten Keten-O,O-acetale der Formel II werden erhalten, indem man ein alpha-Hydroxyacetal der Formel III

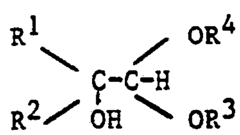

in den entsprechenden Sulfonyl-, Carboxyl-Kohlensäure- oder Dithiokohlensäureester überführt und aus dem so erhaltenen Ester in Gegenwart basischer Katalysatoren die entsprechende Säure abtrennt nach folgendem Schema

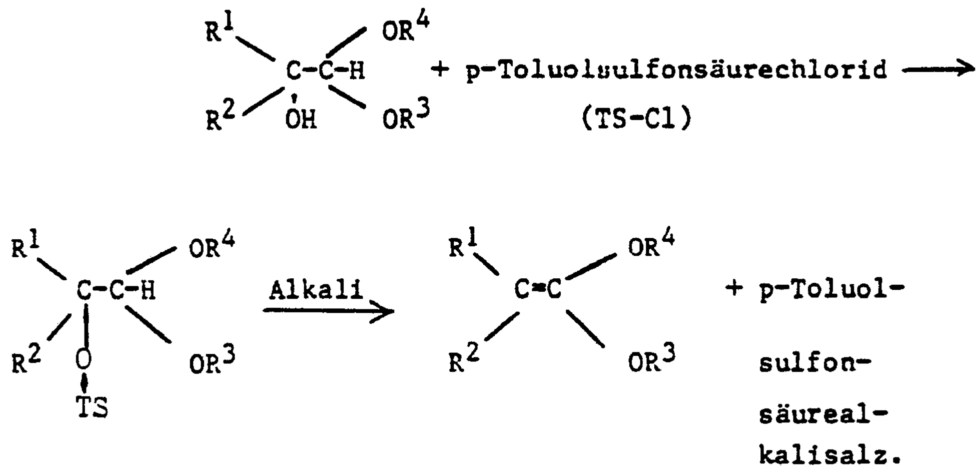


Die Herstellung der Ausgangsprodukte kann entsprechend der folgenden Vorschrift erfolgen.

**Vorschrift**

$a_1$ Tosylierung von 1,1-Bis(2,4-dichlorphenoxy)-3,3-dimethylbutan-2-ol

42,4 g (0,1 mol) 1,1-Bis(2,4-dichlorphenoxy)-3,3-dimethylbutan-2-ol werden in 200 ml abs. Tetrahydrofuran bei -10°C mit der equimolaren Menge einer Lösung von n-Butyllithium in n-Hexan umgesetzt. Anschließend gibt man 19,1 g (0,1 mol) p-Toluolsulfonsäurechlorid zu, läßt auf Raumtemperatur (20°C) auftauen, rührt ca. 1 Stunde nach, destilliert das Lösungsmittel im Vakuum ab, nimmt in Essigester auf und wäscht die organische Phase zweimal mit Wasser. Nach dem Trocknen und einengen der organischen Phase wird das Rohprodukt aus Cyclohexan/Essigester umkristallisiert.

Ausbeute: 51,8 g (95 % d.Th.)
Schmp: 87 bis 88°C
$^1$H-NMR: δ = 1,25 (s, 3 H); 2,4 (s, 3 H); 4,85 (d, 1 H); 6,05 (d, 1 H); 6,5 - 7,9 (m, 10 H)

$a_2$ Tosylierung von 1,1-Bis(4-chlorphenoxy)-3,3-dimethylbutan-2-ol

35,6 g (0,1 mol) 1,1-Bis(4-chlorphenoxy)-3,3-dimethylbutan-2-ol werden in 200 ml abs. THF mit 3 g (0,1 mol) Natriumhydrid (80-%-ig) versetzt und auf 40°C erhitzt. Sobald keine Gasentwicklung mehr stattfindet, gibt man 19,1 g (0,1 mol) p-Toluolsulfonsäurechlorid zu, rührt 1 Stunde bei Raumtemperatur nach, hydrolysiert anschließend mit wenig Wasser und engt im Vakuum ein. Der Rückstand wird in Essigester aufgenommen, zweimal mit Wasser gewaschen, getrocknet und erneut im Vakuum eingeengt. Das verbleibende Rohprodukt wird aus Essigester/Cyclohexan umkristallisiert.

Ausbeute: 41,7 g (82 % d.Th.)
Schmp: 97°C
$^1$H-NMR: δ = 1,25 (s, 9 H); 2,3 (s, 3 H); 4,8 (d, 1 H); 5,8 (d, H); 6,5 - 7,9 (m, 12 H).

Nach der gleichen Vorschrift wurden folgende Verbindungen hergestellt:

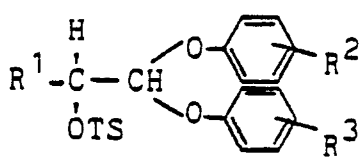

TS:

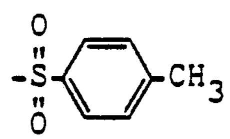

$R^1$ = tertiär-Butyl

| $R^2 = R^3$ | Schmp. in °C | | H-NMR-Daten -Werte in $CDCL_3$ |
|---|---|---|---|
| 2-Cl | 79 | δ = | 1,2 (s, 9 H);<br>2,3 (s, 3 H);<br>4,9 (d, 1 H);<br>6,1 (d, 1 H);<br>6,5 - 7,9 (m, 12 H) |
| 4-Br | 93 - 95 | δ = | 1,2 (s, 9 H);<br>2,3 (s, 3 H);<br>4,8 (d, 1 H);<br>5,8 (d, 1 H);<br>6,5 - 7,9 (m, 12 H) |
| 3,5 $Cl_2$ | 124 - 126 | δ = | 1,2 (s, 9 H);<br>2,4 (s, 3 H);<br>4,85 (d, 1 H);<br>5,8 (d, 1 H);<br>6,6 - 7,9 (m, 10 H); |
| 2,4,5 $Cl_3$ | 116 - 118 | δ = | 1,2 (s, 9 H);<br>2,35 (s, 3 H);<br>4,85 (s, 1 H);<br>5,95 (s, 1 H);<br>6,8 - 7,9 (m, 8 H) |

$b_1$ 1,1-Bis(2,4-dichlorphenoxy)-3,3-dimethylbuten-1

54,6 g (0,1 mol) Tosylat des 1,1-Bis(2,4-dichlorphenoxy)-3,3-dimethylbutan-2-ols werden in 200 ml trockenem Dimethylsulfoxid mit der äquimolaren Menge Kalium-tert.-butanolat versetzt. Nach 30 min. Rühren bei Raumtemperatur hydrolysiert man mit Wasser, extrahiert zweimal mit dem gleichen Volumen Essigsäureethylester, trocknet und destilliert das Lösungsmittel im Vakuum ab.

Ausbeute: 38,57 g (95 % d.Th.)
Sdp: 175 - 176-°C/0,5 mbar
$^1$H-NMR: $\delta$ = 1,2 (s, 9 H); 4,85 (s, 1 H); 7,73 (m, 6 H).

$b_2$ 1,1-Bis(4-chlorphenoxy)-3,3-dimethylbuten-1

47,8 g (0,1 mol) Tosylat des 1,1-Bis(4-chlorphenoxy)-3,3-dimethylbutan-2-ols werden in 200 ml trockenem Dimethylsulfoxtd mit 39 g (0,5 mol) Natrtumsulfid versetzt und so lange bet 120°C gerührt, bis keine Ausgangsverbtndung mtttels Hochdruckflüssigkeitschromatographie (HPLC) mehr nachweisbar ist. Anschließend kühlt man ab, versetzt mit Wasser und extrahiert zweimal mit dem gleichen Volumen Essigester, trocknet und destilliert das Lösungsmittel im Vakuum ab.

Ausbeute: 25,3 g (75 % d.Th.)
$^1$H-NMR: $\delta$ = 1,2 (s, 9 H); 4,8 (s, 1 H); 6,8 - 7,4 (m, 8 H).

Nach dem gleichen Verfahren wurden folgende weitere Beispiele hergestellt:

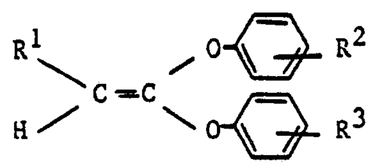

| $R^1 = C(CH_3)_3$ | $R^2 = R^3$ | | H-NMR-Daten-Werte in $CDCl_3$ |
|---|---|---|---|
| | 2-Cl | $\delta$ = | 1,2 (s, 9 H); 4,8 (s, 1 H); 6,8 - 7,4 (m, 8 H) |
| | 4-Br | $\delta$ = | 1,15 (s, 9 H); 4,8 (s, 1 H); 6,7 - 7,5 (m, 8 H) |
| | 3,5-$Cl_2$ | $\delta$ = | 1,15 (s, 9 H); 4,95 (s, 1 H); 6,8 - 7,1 (m, 6 H) |
| | 2,4,5-$Cl_3$ | $\delta$ = | 1,2 (s, 9 H); 4,95 (s, 1 H); 7,25 - 7,5 (m, 4 H) |

Es sind im Prinzip Austauschreaktionen bekannt, die dem erfindungsgemäßen Verfahren ähnlich sind. Es handelt sich dabei aber fast ausschließlich um Reaktionen an aktivierten Keten-O,O-acetalen. Das sind Keten-O,O-acetale, die in β-Stellung elektronenziehende Substituenten wie -CHO, -COOR, -$SO_2$, -CN etc. tragen (vgl. z. B. H.D. Stachel, B. 93, 1059 (1960), US-2 883 368 C.A. 53, 18872 (1959)). Zweitens führt ihre Umsetzung mit Aminen aber immer zu Gemischen aus Keten-O,N- und Keten-N,N-acetalen, die in der Regel schwierig zu trennen sind.

Um so überraschender ist es, daß sich Keten-O,O-acetale mit Phenolresten als OR-Reste in nahezu quantitativer Ausbeute und hoher Stereoselektivität ohne nennenswerte Bildung von Keten-N,N-acetalen mit Heterocyclen z. B. Triazol oder Imidazol zu Keten-O,N-acetalen umsetzen lassen.

Für die praktische Durchführung des erfindungsgemäßen Verfahrens legt man z. B. das Keten-O,O-acetal zusammen mit dem Amin in stöchiometrischen Mengen vor und erhitzt die Mischung unter guter Durchmischung (zum Teil bilden sich Zweiphasensysteme) auf Reaktionstemperatur.

Diese liegt zwischen 100 und 250°C, vorzugsweise zwischen 180 und 200°C. Der Beginn und Verlauf der Reaktion läßt sich durch die zunehmende Homogenisierung der Mischung und Phenolabspaltung verfolgen. Zweckmäßig entnimmt man in bestimmten Zeitabständen Proben aus der Reaktionsmischung und kontrolliert den Reaktionsablauf mittels Gaschromatographie (GC) oder Hochdruckflüssigkeitschromatographie (HPLC).

Man bricht die Reaktion ab, sobald keine Ausgangsverbindung mehr vorliegt. Die Aufarbeitung erfolgt üblicherweise durch Extraktion des abgespaltenen Phenols mit einem wäßrigen basischen Extraktionsmittel, z. B. Natronlauge oder Kalilauge, vorzugsweise in Konzentrationen zwischen 5 und 30 Gew.-% in Wasser aus der organischen Lösung des Endproduktes. Als organische Lösungsmittel kommen aliphatische Kohlenwasserstoffe, Ether oder Ester sowie aromatische Kohlenwasserstoffe wie Toluol oder o, m, p-Xylol in Betracht. Das Endprodukt kann aber auch als quartäres Ammoniumsalz vorzugsweise mit der wäßrigen Lösung einer starken anorganischen Säure wie Chlorwasserstoffsäure, Bromwasserstoffsäure, Schwefelsäure,

Salpeter- oder Perchlorsäure aus der organischen Phase extrahiert werden. Nach Neutralisation der Säure kann dann das freie Keten-O,N-acetal aus der wäßrigen Phase abgetrennt werden.

**Beispiel**

Herstellung von Z-1-(1,2,4-Triazol-1-yl)-1-(2,4-dichlorphenoxy)-3,3-dimethylbuten-1

30,4 g (0,075 mol) 1,1-Bis(2,4-dichlorphenoxy)-3,3-dimethylbuten-1 und 5,2 g (0,075 mol) Triazol werden zusammen auf 180 - 185° C erhitzt. Sobald die Reaktionsmischung homogen erscheint und sich rötlich-braun verfärbt hat (nach etwa 2 - 3 Stunden), wird mittels HPLC der Gehalt an Ausgangsverbindung bestimmt. Hat die Reaktion den gewünschten Umsetzungsgrad erreicht, kühlt man ab, nimmt in 200 ml Hexan/Essigester (1 : 1) auf, wäscht die Lösung 2- bis 3-mal mit dem gleichen Volumen Natronlauge (5 %), trocknet mit $Na_2SO_4$ und destilliert das Lösungsmittel im Vakuum ab. Man erhält 22,2 g Keten-O,N-acetal. Isomerenreinheit: Z-Isomeres 85 % bestimmt mittels $^1H$-NMR und HPLC.

In entsprechender Weise wurden die folgenden Verbindungen hergestellt.

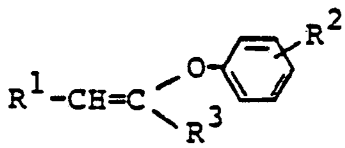

| Nr. | $R^1$ | $R^2$ | $R^3$ | Phys. Daten $^1H$-NMR<br>Reinheit des δ-Werte in<br>Isomeren $CDCl_3$ |
|---|---|---|---|---|
| 1 | $(CH_3)_3C$- | 2,4-$Cl_2$ | 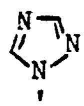 | 85 % Z-Isomeres<br>δ = 1,25 (s, 9H),<br>5,85 (s, 1H), 6,7 - 7 (m, 3H),<br>7,85 (s, 1H), 8,1 (s, 1H) |
| 2 | $(CH_3)_3C$- | 4-Cl | 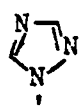 | 90 % Z-Isomeres<br>δ = 1,25 (s, 9H),<br>5,9 (s, 1H), 6,8 - 7,4 (m, 4H),<br>7,95 (s, 1H), 8,2 (s, 1H) |
| 3 | $(CH_3)_3C$- | 2-Cl | 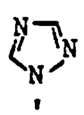 | 90 % Z-Isomeres<br>δ = 1,15 (s, 9H),<br>5,9 (s, 1H), 6,75 - 7,5 (m, 4H),<br>7,9 (s, 1H), 8,15 (s, 1H) |
| 4 | $(CH_3)_3C$- | 4-Cl | 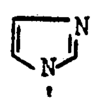 | 80 % E-Isomeres<br>δ = 1,25 (s, 9H), 5,25 (s, 1H),<br>6,7 - 7,7 (m, 7H) |
| 5 | $(CH_3)_3C$- | 2,4-$Cl_2$ | 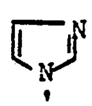 | 75 % Z-Isomeres<br>δ = 1,3 (s, 9H), 5,4 (s, 1H),<br>6,8 - 7,8 (m, 6H) |
| 6 | $(CH_3)_3C$- | 2-Cl | 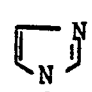 | 80 % Z-Isomeres<br>δ = 1,25 (s, 9H),<br>5,35 (s, 1H), 6,7 - 7,8 (m, 7H) |
| 7 | $(CH_3)_3C$- | 4Cl | 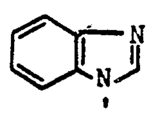 | 80 % Z-Isomeres<br>δ = 1,35 (s, 9H),<br>5,4 (s, 1H), 6,8 - 8,1 (m, 9H) |

**Patentansprüche**

1. Verfahren zur Herstellung von weitgehend reinen E- oder Z-Isomeren von Keten-O,N-acetalen der Formel I

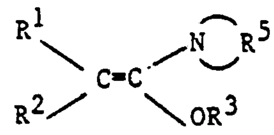

I

in der
$R^1$ einen tertiären Alkylrest mit 4 bis 6 C-Atonen
$R^2$ Wasserstoff,
$R^3$ einen gegebenenfalls durch Halogen, Alkoxy, Alkyl, Phenyl, Phenoxy, Cyano, Nitro, Trifluormethyl, substituierten Phenylrest und

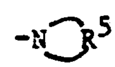

einen Triazol-, Pyrazol-, Benzimidazol oder Imidazolrest bedeutet, dadurch gekennzeichnet, daß man ein Keten-O,O-acetal der Formel II

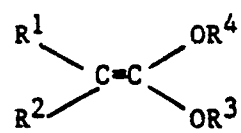

II

in der
$R^1$, $R^2$ und $R^3$ die oben genannten Bedeutungen haben und
$R^4$ einen gegebenenfalls durch Halogen, Alkoxy, Alkyl, Phenyl oder Phenoxy, Cyano, Nitro, Trifluormethyl substituierten Phenylrest
bedeutet, bei einer Temperatur von 100 bis 250°C mit der stöchiometrischen Menge oder bis zu 10 % mehr über oder Unterschuß einer Verbindung der Formel

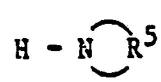

umsetzt, in der

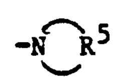

die oben genannten Bedeutungen hat.

2. Verfahren nach Anspruch 1, dadurch gekennzeichnet, daß man die Umsetzung in der Schmelze der Ausgangsprodukte der Formel II und der Verbindung

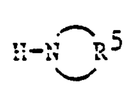

durchführt.

3. Verfahren nach Anspruch 1, dadurch gekennzeichnet, daß man die Umsetzung mit stöchiometrischen Mengen der Verbindungen der Formeln II und

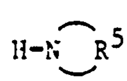

durchführt.

4. Verfahren nach Anspruch 2, dadurch gekennzeichnet, daß man die Umsetzung bei einer Temperatur von 180 bis 200°C durchführt.

**Claims**

1. A process for the preparation of a substantially pure E- or Z- isomer of a ketene O,N-acetal of the formula I

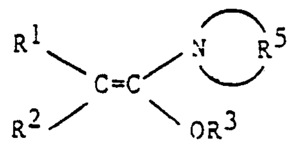

I

where
$R^1$ is tertiary alkyl of 4 to 6 carbon atoms,
$R^2$ is hydrogen,
$R^3$ is phenyl which is unsubstituted or substituted by halogen, alkoxy, alkyl, phenyl, phentoxy, cyano, nitro or trifluoromethyl, and

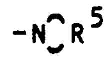

is a triazole, pyrazole, benzimidazole or imidazole radical, wherein a ketene O,O-acetal of the formula

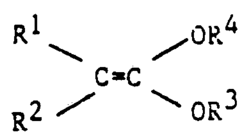

II

where
$R^1$, $R^2$ and $R^3$ have the above meanings and
$R^4$ is phenyl which is unsubstituted or substituted by halogen, alkoxy, alkyl, phenyl, phenoxy, cyano, nitro or trifluoromethyl, is reacted with a stoichiometric, or a not more than 10 % super- or substoichiometric, amount of a compound of the formula

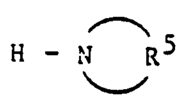

where

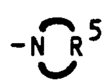

has the above meanings, at from 100 to 250° C.

2. A process as claimed in claim 1, wherein the reaction is carried out in the melt of the starting product of the formula II and the compound

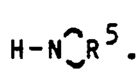

3. A process as claimed in claim 1, wherein the reaction is carried out with stoichiometric amounts of the compound of the formula II and

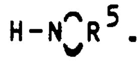

4. A process as claimed in claim 2, wherein the reaction is carried out at from 180 to 200°C.

**Revendications**

1. Procédé de préparation d'isomères E ou Z extrêmement purs de cétène-O,N-acétals de formule I

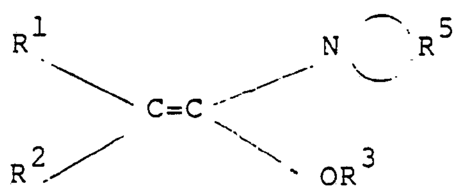

I

dans laquelle
$R^1$ représente un reste alkyle tertiaire de 4 à 6 atomes C
$R^2$, hydrogène,
$R^3$, un reste phényle, éventuellement substitué par halogène, alcoxy, alkyle, phényle, phénoxy, cyano, nitro, trifluorométhyle, et

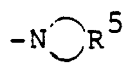

un reste triazole, pyrazole, benzimidazole ou imidazole caractérisé par le fait que l'on fait réagir, à température de 100 à 250°C un cétène -O,O-acétal de formule II

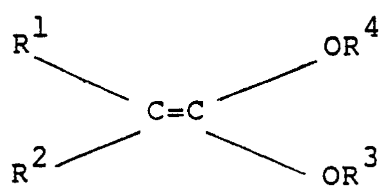

II

dans laquelle
$R^1$, $R^2$ et $R^3$ ont les significations indiquées plus haut et
$R^4$ représente un reste phényle, éventuellement substitué par halogène, alcoxy, alkyle, phényle, phénoxy, cyano, nitro, trifluorométhyle,
avec la quantité stoechiométrique,ou jusqu'à 10 %, d'un composé de formule

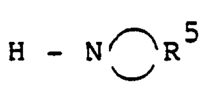

dans laquelle

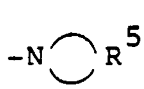

a les signfications indiquées plus haut.

2. Procédé selon la revendication 1, caractérisé par le fait que l'on effectue la réaction dans la masse fondue du produit de départ de formule II et du composé

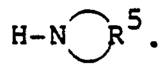

3. Procedé selon la revendication 1, caractérisé par le fait que l'on effectue la réaction avec une quantité stoechiométrique de composés de formule II et

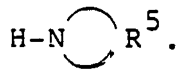

4. Procédé selon la revendication 2, caractérisé par le fait que l'on effectue la réaction à une température de 180 à 200° C.